(19) Europäisches Patentamt
European Patent Office
Office européen des brevets

(11) **EP 3 372 997 B1**

(12) **EUROPÄISCHE PATENTSCHRIFT**

(45) Veröffentlichungstag und Bekanntmachung des Hinweises auf die Patenterteilung:
**01.01.2020 Patentblatt 2020/01**

(51) Int Cl.:
**G01N 27/02** *(2006.01)*  **G01N 27/08** *(2006.01)*
**G01R 27/22** *(2006.01)*  **G01R 27/26** *(2006.01)*
**C02F 1/00** *(2006.01)*  **G01N 33/18** *(2006.01)*

(21) Anmeldenummer: **18160990.0**

(22) Anmeldetag: **09.03.2018**

(54) **VERFAHREN ZUM BETREIBEN EINES INDUKTIVEN LEITFÄHIGKEITSMESSGERÄTS UND DIESBEZÜGLICHES INDUKTIVES LEITFÄHIGKEITSMESSGERÄT**

METHOD FOR OPERATING AN INDUCTIVE CONDUCTIVITY SENSOR AND RELATED INDUCTIVE CONDUCTIVITY SENSOR

PROCÉDÉ DE FONCTIONNEMENT D'UN APPAREIL DE MESURE DE CONDUCTIVITÉ INDUCTIF ET APPAREIL DE MESURE DE CONDUCTIVITÉ INDUCTIF CORRESPONDANT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **09.03.2017 DE 102017104994**

(43) Veröffentlichungstag der Anmeldung:
**12.09.2018 Patentblatt 2018/37**

(73) Patentinhaber: **Krohne Messtechnik GmbH**
**47058 Duisburg (DE)**

(72) Erfinder: **GLASMACHERS, Holger**
**44795 Bochum (DE)**

(74) Vertreter: **Gesthuysen Patent- und Rechtsanwälte**
**Patentanwälte**
**Huyssenallee 100**
**45128 Essen (DE)**

(56) Entgegenhaltungen:
**DE-A1-102009 026 403     DE-B3-102016 104 549**
**GB-A- 1 085 557          US-A- 3 491 287**

**Beschreibung**

Die Erfindung betrifft zum einen ein Verfahren zum Betreiben eines induktiven Leitfähigkeitsmessgeräts. Das induktive Leitfähigkeitsmessgerät weist zur Ausführung des Verfahrens eine Sendespule, eine Empfangsspule und eine Abschlussimpedanzeinrichtung auf. Dabei weist die Sendespule einen Sendespulenanschluss, die Empfangsspule einen Empfangsspulenanschluss und die Abschlussimpedanzeinrichtung eine Abschlussimpedanz auf. Die Empfangsspule ist durch die Abschlussimpedanzeinrichtung elektrisch abgeschlossen und die Sendespule und die Empfangsspule sind durch ein elektrisch leitfähiges Medium induktiv miteinander gekoppelt.

Die Erfindung betrifft zum anderen auch ein induktives Leitfähigkeitsmessgerät. Das induktive Leitfähigkeitsmessgerät weist ebenfalls eine Sendespule, eine Empfangsspule und eine Abschlussimpedanzeinrichtung und zusätzlich noch eine Steuerungseinrichtung auf. Dabei weist die Sendespule einen Sendespulenanschluss, die Empfangsspule einen Empfangsspulenanschluss und die Abschlussimpedanzeinrichtung eine Abschlussimpedanz auf. Die Empfangsspule ist durch die Abschlussimpedanzeinrichtung elektrisch abgeschlossen. Im Betrieb des induktiven Leitfähigkeitsmessgeräts sind die Sendespule und die Empfangsspule durch ein elektrisch leitfähiges Medium induktiv miteinander gekoppelt.

Im Gegensatz zu konduktiven Leitfähigkeitsmessgeräten können induktive Leitfähigkeitsmessgeräte auch in aggressiven und korrosiven Medien wie beispielsweise industriellen Abwässern, Meerwasser und sauren Lösungen eingesetzt werden. Das ist möglich, weil sowohl die Sendespule als auch die Empfangsspule im Gegensatz zu den Elektroden eines konduktiven Leitfähigkeitsmessgeräts nicht in unmittelbarem Kontakt mit einem Medium stehen müssen, sondern von einem gegen aggressive und korrosive Medien resistenten Gehäuse umgeben sein können, ohne dass die Funktionsfähigkeit beeinträchtigt ist. Da bei Vorhandensein eines Gehäuses kein unmittelbarer Kontakt mit aggressiven und korrosiven Medien gegeben ist, zeichnen sich induktive Leitfähigkeitsmessgeräte durch eine lange Lebensdauer und durch eine weitgehende Wartungsfreiheit im Vergleich zu konduktiven Leitfähigkeitsmessgeräten aus. Durch ein Gehäuse sind sie darüber hinaus auch für hygienische Anwendungen in Prozessen der Branchen Lebensmittel, Getränke und Pharmaka geeignet.

Ein Spulenanschluss einer Spule wie zum Beispiel der Sendespulenanschluss der Sendespule oder der Empfangsspulenanschluss der Empfangsspule weist für gewöhnlich zwei elektrische Anschlüsse auf. Bei der Sendespule wird über die elektrischen Anschlüsse des Sendespulenanschlusses ein elektrisches Sendewechselsignal in die Sendespule eingespeist. Die induktive Kopplung der Sendespule und der Empfangsspule miteinander durch das elektrisch leitfähige Medium erfolgt, indem das in die Sendespule eingespeiste Sendewechselsignal in dem Medium Wirbelströme erzeugt und die Wirbelströme in der Empfangsspule ein elektrisches Empfangswechselsignal induzieren. Das Empfangswechselsignal ist an den elektrischen Anschlüssen des Empfangsspulenanschlusses der Empfangsspule in Form einer Spannung oder eines Stroms messbar. Somit bewirkt ein Sendewechselsignal ein Empfangswechselsignal. Ein Wechselsignal wie zum Beispiel das Sendewechselsignal oder das Empfangswechselsignal ist für gewöhnlich im zeitlichen Verlauf sinusförmig oder rechteckig.

Auch die Abschlussimpedanzeinrichtung weist für gewöhnlich zwei elektrische Anschlüsse auf, zwischen welchen die Abschlussimpedanz in Erscheinung tritt. Die Abschlussimpedanz wird somit von der Abschlussimpedanzeinrichtung realisiert und ist als solche eine Eigenschaft der Abschlussimpedanzeinrichtung.

Das elektrische Abschließen der Empfangsspule mit der Abschlussimpedanzeinrichtung erfolgt durch elektrisches Verbinden der elektrischen Anschlüsse des Empfangsspulenanschlusses mit den elektrischen Anschlüssen der Abschlussimpedanzeinrichtung, sodass die Abschlussimpedanzeinrichtung elektrisch parallel zur Empfangsspule geschaltet ist.

Aus dem Stand der Technik ist bekannt, eine elektrische Leitfähigkeit eines Mediums, welches die Sendespule und die Empfangsspule induktiv miteinander koppelt, aus dem Verhältnis einer Amplitude eines Empfangswechselsignals und einer Amplitude eines Sendewechselsignals zu bestimmen. Dabei ist für gewöhnlich die Amplitude des Sendewechselsignals fest vorgegeben und wird die Amplitude des Empfangswechselsignals gemessen. Problematisch bei der Bestimmung einer Amplitude wie der Amplitude des Empfangswechselsignals ist jedoch, dass die Genauigkeit der Bestimmung nicht über den gesamten Wertebereich, den die Amplitude einnehmen kann, konstant ist, was zu Fehlern bei der Bestimmung der elektrischen Leitfähigkeit des Mediums führen kann.

Die US3491287 A offenbart ein induktives Leitfähigkeitsmessgerät mit Sende- und Empfängerspule und einer Abschlussimpedanzeinrichtung. Die Abschlussimpedanzeinrichtung besitzt insbesondere einstellbare Widerstände, um die Impedanz der Empfängerspule verschiedenen Messbereichen anzupassen und die Brückenschaltung auszubalancieren, insbesondere an den Eingängen des Detektors. Insbesondere das Ausbalancieren erfolgt über ein Potentiometer, dessen Arm mit einem Stiftaufnehmer gekoppelt ist, dessen Position die Salinität von Wasser anzeigt.

Die DE102009026403 A1 offenbart ein induktives Leitfähigkeitsmessgerät mit Sende- und Empfängerspule, wobei die Empfängerspule über eine Abschlussimpedanz $R_{Ab}$ abgeschlossen ist. Ferner besitzt die Anordnung einen Kurzschlusspfad mit der Sendespule, über den die die Abschlussimpedanz auch an der Sendeseite (Primärseite) messbar ist. Ist die Abschlussimpedanz $R_{Ab}$ nicht auf der Sendeseite messbar, so deutet dies auf ein gebrochenes Kabel zwischen Empfängerspule und Messschaltung hin.

Die D3 GB 1085557 offenbart ein induktives Leitfähigkeitsmessgerät mit Sende- und Empfängerspule und eine Temperaturkompensationseinrichtung und einer regelbaren Impedanz zwischen Sendetransformator und Empfängertransformator. Die Messung der Leitfähigkeit einer Flüssigkeit erfolgt über eine Brückenschaltung, die über die regelbare Impedanz vor der Messung ausbalanciert wird. Die Messung erfolgt über die Messung einer Frequenzverschiebung eines Oszillators in der Brückenschaltung, oder indem die regelbare Impedanz die Brückenschaltung erneut ausbalanciert. Die Änderung der Impedanz ist dann ebenso ein Indikator für die Leitfähigkeit der Flüssigkeit.

Der Erfindung liegt daher die Aufgabe zugrunde, ein Verfahren zum Betreiben eines induktiven Leitfähigkeitsmessgeräts sowie ein induktives Leitfähigkeitsmessgerät anzugeben, bei dem die Genauigkeit einer Bestimmung einer Leitfähigkeit eines Mediums gegenüber dem Stand der Technik verbessert ist.

Die Erfindung bezieht sich gemäß einer ersten Lehre auf ein Verfahren zum Betreiben eines induktiven Leitfähigkeitsmessgeräts, bei dem die dargelegte Aufgabe gelöst ist. Das erfindungsgemäße Verfahren ist zunächst und im Wesentlichen durch die folgenden Verfahrensschritte gekennzeichnet:

In einem ersten Verfahrensschritt wird eine Soll-Eingangsimpedanz vorgegeben.

In einem zweiten Verfahrensschritt wird eine Eingangsimpedanz am Sendespulenanschluss bestimmt.

In einem dritten Verfahrensschritt wird die Abschlussimpedanz derart eingestellt, dass die Eingangsimpedanz an die Soll-Eingangsimpedanz angepasst ist.

In einem vierten Verfahrensschritt wird dann eine Leitfähigkeit des Mediums unter Verwendung der angepassten Eingangsimpedanz und der eingestellten Abschlussimpedanz bestimmt.

Erfindungsgemäß werden die einzelnen Verfahrensschritte nach Bedarf auch mehrfach ausgeführt. Das gilt insbesondere für den zweiten und dritten Verfahrensschritt, wenn die Eingangsimpedanz mittels eines iteratives Verfahrens durch Variation der Abschlussimpedanz an die Soll-Eingangsimpedanz angepasst wird.

Der Erfindung liegt die Erkenntnis zugrunde, dass die elektrische Leitfähigkeit des Mediums, welches die Sendespule und die Empfangsspule induktiv miteinander koppelt, ein Parameter sowohl in der Eingangsimpedanz als auch in einer Übertragungsfunktion ist, welche die Übertragung von elektrischen Wechselsignalen zwischen dem Sendespulenanschluss und dem Empfangsspulenanschluss beschreibt. Wird zum Beispiel ein vorgegebenes Sendewechselsignal in den Sendespulenanschluss der Sendespule eingespeist und verändert sich die Leitfähigkeit des Mediums, so verändert sich zum einen die Eingangsimpedanz und zum anderen das Empfangswechselsignal am Empfangsspulenanschluss entsprechend. Durch die Einstellung der Abschlussimpedanz, welche wie die Leitfähigkeit ebenfalls ein Parameter sowohl in der Eingangsimpedanz als auch in der Übertragungsfunktion ist, derart, dass die Eingangsimpedanz wieder, also wie vor der Veränderung der Leitfähigkeit, an die Soll-Eingangsimpedanz angepasst ist, wird die Auswirkung der Veränderung der elektrischen Leitfähigkeit des Mediums auf die Eingangsimpedanz kompensiert. Somit ist in der Veränderung der Abschlussimpedanz die Information über die Veränderung der Leitfähigkeit des Mediums enthalten. Dadurch ist es möglich, die Leitfähigkeit des Mediums unter Verwendung der angepassten Eingangsimpedanz und der eingestellten Abschlussimpedanz zu bestimmten.

Eine Impedanz, wie die Soll-Eingangsimpedanz, Eingangsimpedanz und Abschlussimpedanz, weist einen Realteil und/oder einen Imaginärteil auf. Sowohl der Real- als auch der Imaginärteil einer Impedanz können mit passiven elektrischen Bauelementen wie zum Beispiel Widerständen, Spulen und Kondensatoren realisiert werden, wobei eine Eigenschaft eines Widerstands ein Widerstandswert, eine Eigenschaft einer Spule eine Induktivität und eine Eigenschaft eines Kondensators eine Kapazität ist. Demnach trägt ein Widerstandswert zum Realteil und tragen Kapazitäten und Induktivitäten zum Imaginärteil einer Impedanz bei.

Die Eingangsimpedanz am Sendespulenanschluss wird zum Beispiel bestimmt, indem in den Sendespulenanschluss, also an den beiden elektrischen Anschlüssen, ein Sendewechselsignal in Form einer vorgegebenen Sendewechselspannung mit einer Amplitude eingespeist wird, eine durch die Sendewechselspannung bewirkte Amplitude eines Sendewechselstrom durch den Sendespulenanschluss gemessen wird und aus der vorgegebenen Amplitude der Sendewechselspannung und der gemessenen Amplitude des Sendewechselstroms die Eingangsimpedanz bestimmt wird. Alternativ kann die Eingangsimpedanz zum Beispiel auch bestimmt werden, indem in den Sendespulenanschluss ein Sendewechselsignal in Form eines vorgegebenen Sendewechselstroms mit einer Amplitude eingespeist wird, eine durch den Sendewechselstrom bewirkte Amplitude einer Sendewechselspannung über den Sendespulenanschluss gemessen wird und aus der vorgegebenen Amplitude des Sendewechselstroms und der gemessenen Amplitude der Sendewechselspannung die Eingangsimpedanz bestimmt wird.

Die Leitfähigkeit wird für gewöhnlich bestimmt, indem ein elektrischer Widerstandswert des Mediums zwischen der Sende- und der Empfangsspule und dann die Leitfähigkeit des Mediums unter Verwendung des Widerstandswerts und einer Geometrie des Mediums zwischen Sende- und Empfangsspule bestimmt wird.

Das erfindungsgemäße Verfahren hat gegenüber aus dem Stand der Technik bekannten Verfahren den Vorteil, dass die Bestimmung der Leitfähigkeit des Mediums genauer ist. Die höhere Genauigkeit ergibt sich insbesondere daraus, dass eine Veränderung der Eingangsimpedanz kompensiert und nicht, wie aus dem Stand der Technik bekannt, gemessen wird. Dadurch ist der Wertebereich, den die zu messenden Amplituden einnehmen, geringer als der Wertebereich, den die aus dem Stand der Technik bekannten zu messenden Amplituden einnehmen, sodass Ungenauigkeiten bei der Messung von Amplituden geringer sind. Der technische Aufwand zur Umsetzung des Verfahrens ist vergleichbar mit dem technischen Aufwand zur Umsetzung des aus dem Stand der Technik bekannten Verfahrens.

In einer Ausgestaltung des Verfahrens zum Betreiben des induktiven Leitfähigkeitsmessgeräts ist vorgesehen, dass die Eingangsimpedanz derart an die Soll-Eingangsimpedanz angepasst wird, dass ein Realteil der Eingangsimpedanz einem Realteil der Soll-Eingangsimpedanz entspricht. Für gewöhnlich weist die Eingangsimpedanz einen Realteil und einen Imaginärteil auf. Dabei trägt die Leitfähigkeit des Mediums zum Realteil und Induktivitäten der Sendespule und der Empfangsspule zum Imaginärteil bei. Somit ist es grundsätzlich ausreichend, wenn der Realteil der Eingangsimpedanz dem Realteil der Soll-Eingangsimpedanz entspricht.

In einer weiteren Ausgestaltung des Verfahrens ist vorgesehen, dass die Eingangsimpedanz derart an die Soll-Eingangsimpedanz angepasst wird, dass die Eingangsimpedanz der Soll-Eingangsimpedanz entspricht. Somit entspricht dann nicht nur der Realteil der Eingangsimpedanz dem Realteil der Soll-Eingangsimpedanz, sondern auch der Imaginärteil der Eingangsimpedanz dem Imaginärteil der Soll-Eingangsimpedanz. Wenn zum einen die Realteile und zum anderen auch die Imaginärteile einander entsprechen, ist die Genauigkeit der Bestimmung der Leitfähigkeit des Mediums vergrößert im Vergleich zu einem Verfahren, bei welchem nur die Realteile einander entsprechen. Zwei Realteile oder zwei Imaginärteile entsprechen insbesondere dann einander, wenn sie den gleichen Betrag aufweisen.

Die Erfindung bezieht sich gemäß einer zweiten Lehre auch auf ein induktives Leitfähigkeitsmessgerät, bei dem die dargelegte Aufgabe gelöst ist. Das erfindungsgemäße induktive Leitfähigkeitsmessgerät ist zunächst und im Wesentlichen dadurch gekennzeichnet, dass eine Soll-Eingangsimpedanz in der Steuerungseinrichtung gespeichert ist, dass die Abschlussimpedanz der Abschlussimpedanzeinrichtung einstellbar ist und dass die Steuerungseinrichtung derart ausgebildet ist, eine Eingangsimpedanz am Sendespulenanschluss zu bestimmen, die Abschlussimpedanz derart einzustellen, dass die Eingangsimpedanz an die Solleingangsimpedanz angepasst ist und eine Leitfähigkeit des Mediums unter Verwendung der angepassten Eingangsimpedanz und der eingestellten Abschlussimpedanz zu bestimmen.

Eine Ausgestaltung des erfindungsgemäßen induktiven Leitfähigkeitsmessgeräts sieht vor, dass die Steuerungseinrichtung zur Ausführung eines der beschriebenen Verfahren ausgebildet ist. Weiterhin ist die Steuerungseinrichtung für gewöhnlich dazu ausgebildet, Sendewechselsignale zu erzeugen und in die Sendespule zu speisen und Empfangswechselsignale zu messen.

In einer weiteren Ausgestaltung des induktiven Leitfähigkeitsmessgeräts ist vorgesehen, dass die Abschlussimpedanzeinrichtung einen Stromsensor zur Messung eines Empfangsspulenstroms durch den Empfangsspulenanschluss und eine gesteuerte Spannungsquelle zur Einstellung einer Empfangsspulenspannung über den Empfangsspulenanschluss aufweist. Eine gesteuerte Spannungsquelle ist eine Spannungsquelle, bei welcher durch eine Signalquelle eine Spannung, wie die Empfangsspulenspannung, der Spannungsquelle einstellbar ist. Weiter ist die Steuerungseinrichtung ausgebildet, den Empfangsspulenstrom mit dem Stromsensor zu bestimmen und die Empfangsspulenspannung mit der gesteuerten Spannungsquelle derart einzustellen, dass die Abschlussimpedanz eingestellt ist. Somit stellt die Abschlussimpedanzeinrichtung eine aktive Impedanz dar.

In einer Weiterbildung der vorstehenden Ausgestaltung ist vorgesehen, dass der Stromsensor ein Shunt-Widerstand ist und die gesteuerte Spannungsquelle einen Operationsverstärker zur Bereitstellung der Empfangsspulenspannung aufweist. Ein Shunt-Widerstand ist ein niederohmiger elektrischer Widerstand zur Messung von elektrischen Strömen. Der Operationsverstärker weist dabei eine derartige externe Beschaltung auf, dass er eine Spannungsquelle darstellt.

In einer Weiterbildung der vorstehenden Weiterbildung ist vorgesehen, dass die gesteuerte Spannungsquelle einen Signalgenerator zur Steuerung des Operationsverstärkers aufweist. Somit ist der Signalgenerator die Signalquelle, welche die Empfangsspulenspannung, die von dem Operationsverstärker bereitgestellt wird, dem Operationsverstärker vorgibt.

In einer Weiterbildung der vorstehenden Weiterbildung ist weiterhin vorgesehen, dass der Signalgenerator ausgebildet ist, ein wertkontinuierliches Signal oder ein pulsweitenmoduliertes Signal zur Ansteuerung des Operationsverstärkers zu erzeugen.

Die vorstehende Ausgestaltung mit dem Stromsensor und der gesteuerten Spannungsquelle, insbesondere in Verbindung mit den Weiterbildungen dieser Ausgestaltung, realisiert eine kostengünstige aktive Abschlussimpedanzeinrichtung.

Die Ausführungen zum Verfahren zum Betreiben eines induktiven Leitfähigkeitsmessgeräts gelten entsprechend für das induktive Leitfähigkeitsmessgerät und umgekehrt.

Im Einzelnen ist eine Vielzahl von Möglichkeiten gegeben, das erfindungsgemäße Verfahren zum Betreiben eines induktiven Leitfähigkeitsmessgeräts und das induktive Leitfähigkeitsmessgerät auszugestalten und weiterzubilden. Dazu wird verwiesen sowohl auf die den unabhängigen Patentansprüchen nachgeordneten Patentansprüchen als auch auf die nachfolgende Beschreibung eines bevorzugten Ausführungsbeispiels in Verbindung mit der Zeichnung. In der Zeichnung zeigt schematisch und abstrahiert

Fig. 1    ein Ausführungsbeispiel eines induktiven Leitfähigkeitsmessgeräts,

Fig. 2    ein teilweises elektrisches Ersatzschaltbild des induktiven Leitfähigkeitsmessgeräts aus Fig. 1,

Fig. 3    ein teilweises elektrisches Ersatzschaltbild der Abschlussimpedanzeinrichtung aus Fig. 2,

Fig. 4    ein teilweises elektrisches Ersatzschaltbild der gesteuerten Spannungsquelle aus Fig. 3 und

Fig. 5    einen Ablaufplan eines Ausführungsbeispiels eines Verfahrens zum Betreiben des induktiven Leitfähigkeitsmessgeräts aus Fig. 1.

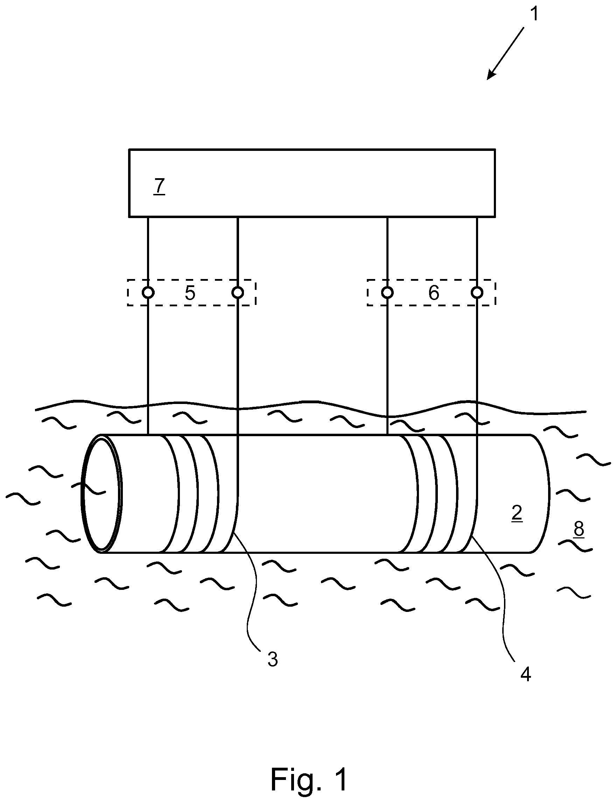

Fig. 1 zeigt das induktive Leitfähigkeitsmessgerät 1. Das induktive Leitfähigkeitsmessgerät 1 weist den hohlzylinderförmigen Träger 2 auf, auf welchem die Sendespule 3 und die Empfangsspule 4 angeordnet sind. Die Sendespule 3 und die Empfangsspule 4 sind auf dem hohlzylinderförmigen Träger 2 angeordnet, indem diese um den hohlzylinderförmigen Träger 2 gewickelt sind, wobei die Sendespule 3 die Anzahl $N_1$ und die Empfangsspule 4 die Anzahl $N_4$ Windungen aufweist. Weiter weist die Sendespule 3 den elektrischen Sendespulenanschluss 5 und die Empfangsspule 4 den elektrischen Empfangsspulenanschluss 6 auf. Das induktive Leitfähigkeitsmessgerät 1 weist darüber hinaus auch die Steuerungseinrichtung 7 auf, die zur Steuerung der Sendespule 3 und der Empfangsspule 4 ausgebildet ist, weshalb die Steuerungseinrichtung 7 auch zum einen mit dem Sendespulenanschluss 5 der Sendespule 3 und zum anderen mit dem Empfangsspulenanschluss 6 der Empfangsspule elektrisch verbunden ist. In der Steuerungseinrichtung 7 ist darüber hinaus eine Soll-Eingangsimpedanz gespeichert.

Der hohlzylinderförmige Träger 2 mit der Sendespule 3 und der Empfangsspule 4 ist in das Medium 8 eingetaucht und das induktive Leitfähigkeitsmessgerät 1 ist im Betrieb. Das Medium 8 umgibt den hohlzylinderförmigen Träger 2 und ist auch in seinem Innenraum vorhanden. Das Medium 8 ist elektrisch leitfähig und koppelt dadurch die Sendespule 3 und die Empfangsspule 4 induktiv miteinander. Da es sich um eine abstrahierte schematische Darstellung des induktiven Leitfähigkeitsmessgeräts 1 handelt, ist ein für gewöhnlich vorhandenes Gehäuse, welches einen unmittelbaren Kontakt der Sendespule 3 und der Empfangsspule 4 mit dem Medium 8 verhindert, hier nicht dargestellt. Durch die Vermeidung eines Kontakts der Sendespule 3 und der Empfangsspule 4 mit dem Medium 8 ist es möglich, das induktive Leitfähigkeitsmessgerät 1 im Gegensatz zu konduktiven Leitfähigkeitsmessgeräten auch in aggressiven und korrosiven Medien wie beispielsweise industriellen Abwässern, Meerwasser und sauren Lösungen einzusetzen, ohne dass die Funktionsfähigkeit des induktiven Leitfähigkeitsmessgeräts 1 eingeschränkt ist. Durch das Gehäuse ist es darüber hinaus auch für hygienische Anwendungen in Prozessen der Branchen Lebensmittel, Getränke und Pharmaka geeignet.

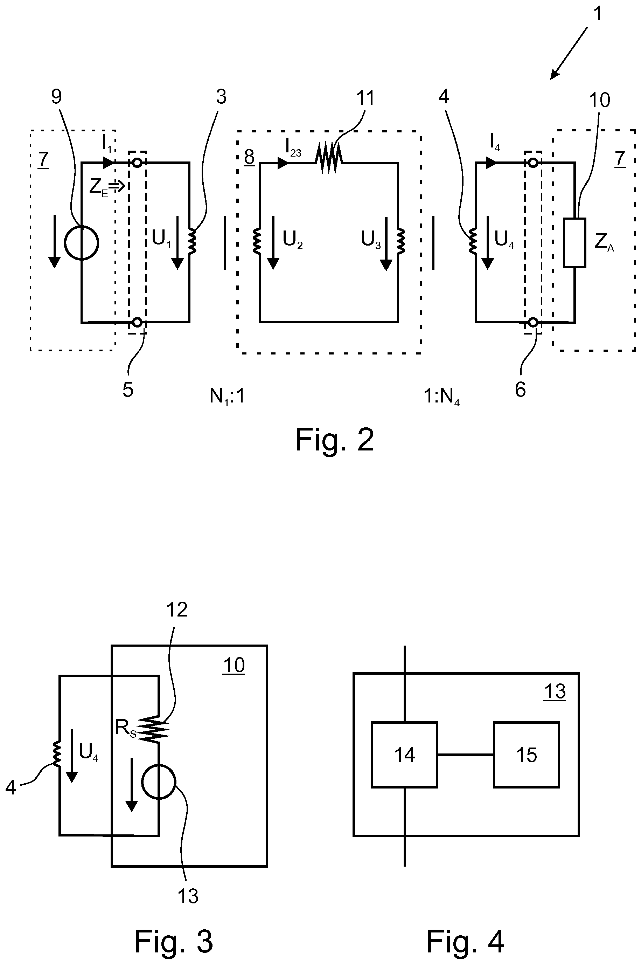

Fig. 2 zeigt ein teilweises elektrisches Ersatzschaltbild zum einen des induktiven Leitfähigkeitsmessgeräts 1 und zum anderen des elektrisch leitfähigen Mediums 8.

Das Ersatzschaltbild des induktiven Leitfähigkeitsmessgeräts 1 weist die Sendespule 3, den Sendespulenanschluss 5 und die Sendewechselspannungsquelle 9 auf, wobei die Sendewechselspannungsquelle 9 derart mit dem Sendespulenanschluss 5 verbunden ist, dass die Sendewechselspannungsquelle 9 und die Sendespule 3 elektrisch parallel geschaltet sind. Weiter weist das Ersatzschaltbild des induktiven Leitfähigkeitsmessgeräts 1 die Empfangsspule 4, den Empfangsspulenanschluss 6 und die Abschlussimpedanzeinrichtung 10 auf, welche in diesem Ausführungsbeispiel Teil der Steuerungseinrichtung 7 ist. Die Abschlussimpedanzeinrichtung 10 ist derart mit dem Empfangsspulenanschluss 6 verbunden, dass die Abschlussimpedanzeinrichtung 10 und die Empfangsspule 4 elektrisch parallel geschaltet sind.

Das Ersatzschaltbild des elektrisch leitfähigen Mediums 8 weist den Mediumwiderstand 11 mit dem Mediumwiderstandswert $R_W$ auf, welcher den elektrischen Widerstand des Mediums 8 zwischen der Sendespule 3 und der Empfangsspule 4 repräsentiert.

Die Sendewechselspannungsquelle 9 ist ausgebildet, eine von der Steuerungseinrichtung 7 vorgegebene sinusförmige Sendespulenspannung $U_1$ zu erzeugen und die Steuerungseinrichtung 7 ist ausgebildet den Sendespulenstrom $I_1$ zu messen. Somit ist die Sendespulenspannung $U_1$ eine Sendewechselspannung und der Sendespulenstrom $I_1$ ein Sendewechselstrom. Aufgrund der Parallelschaltung von Sendewechselspannungsquelle 9 und Sendespule 3 liegt die Sendespulenspannung $U_1$ auch über der Sendespule 3 an und fließt der Sendespulenstrom $I_1$ durch die Sendespule 3. Weiter ist Steuerungseinrichtung 7 ausgebildet, aus der Sendespulenspannung $U_1$, die für gewöhnlich auch von der Steuerungseinrichtung 7 gemessen wird, und dem Sendespulenstrom $I_1$ die Eingangsimpedanz $Z_E = U_1 / I_1$ am Sendespulenanschluss 5 zu bestimmen.

Die induktive Kopplung der Sendespule 3 und der Empfangsspule 4 miteinander durch das elektrisch leitfähige Medium 8 erfolgt, indem das in die Sendespule 3 eingespeiste Sendewechselsignal, welches durch die Sendespulenspannung $U_1$ und den Sendespulenstrom $I_1$ bestimmt ist, in dem Medium 8 Wirbelströme erzeugt und die Wirbelströme in der Empfangsspule 4 ein Empfangswechselsignal induzieren, welches durch die Empfangsspulenspannung $U_4$ und den Empfangsspulen-

strom $I_4$ bestimmt ist. Die Empfangsspulenspannung $U_4$ liegt über der Empfangsspule 4 an und der Empfangsspulenstrom $I_4$ fließt durch die Empfangsspule 4. Aufgrund der Parallelschaltung von Empfangsspule 4 und Abschlussimpedanzeinrichtung 10 liegt die Empfangsspulenspannung $U_4$ auch über der Abschlussimpedanzeinrichtung 10 an und fließt der Empfangsspulenstrom $I_4$ auch durch die Abschlussimpedanzeinrichtung 10. Die Empfangsspulenspannung $U_4$ und der Empfangsspulenstrom $I_4$ sind durch die Abschlussimpedanz $Z_A = U_4 / I_4$ bestimmt. Die Erzeugung der Wirbelströme im Medium 8 durch das Sendewechselsignal erfolgt durch eine transformatorische Kopplung zwischen der Sendespule 3 und dem Medium 8 gemäß $U_1 = N_1 \cdot U_2$ und die transformatorische Kopplung zwischen dem Medium 8 und der Empfangsspule 4 erfolgt gemäß $U_4 = U_3 \cdot N_4$.

Ein teilweises elektrisches Ersatzschaltbild der Abschlussimpedanzeinrichtung 10 ist in Fig. 3 dargestellt. Die Abschlussimpedanz $Z_A$ der Abschlussimpedanzeinrichtung 10 ist einstellbar und die Steuerungseinrichtung 7 ist ausgebildet, die Abschlussimpedanz $Z_A$ der Abschlussimpedanzeinrichtung 10 einzustellen. Somit steuert die Steuerungseinrichtung 7 die Abschlussimpedanzeinrichtung 10 Die Abschlussimpedanzeinrichtung 10 weist zur Einstellung der Abschlussimpedanz einen Stromsensor 12 zur Messung des Empfangsspulenstroms $I_4$ durch den Empfangsspulenanschluss 6 und eine gesteuerte Spannungsquelle 13 zur Einstellung der Empfangsspulenspannung $U_4$ über den Empfangsspulenanschluss auf. Die Steuerungseinrichtung 7 ist zur Einstellung der Abschlussimpedanz $Z_A$ ausgebildet, den Empfangsspulenstrom $I_4$ mit dem Stromsensor 12 zu bestimmen und die Empfangsspulenspannung $U_4$ derart einzustellen, dass die Abschlussimpedanz $Z_A$ eingestellt ist. Somit wird eine aktive Abschlussimpedanz $Z_A$ durch die Abschlussimpedanzeinrichtung 10 und die Steuerungseinrichtung 7 implementiert. Im vorliegenden Ausführungsbeispiel ist der Stromsensor 12 ein Shunt-Widerstand und weist die gesteuerte Spannungsquelle 13 den in Fig. 4 dargestellten Operationsverstärker 14 zur Bereitstellung der Empfangsspulenspannung $U_4$ und den Signalgenerator 15 zur Steuerung des Operationsverstärkers 14 auf. Dabei ist die Steuerungseinrichtung 7 ausgebildet, den Signalgenerator 15 zu steuern. Der Signalgenerator 15 ist im vorliegenden Ausführungsbeispiel ausgebildet, ein wertkontinuierliches Signal zur Ansteuerung des Operationsverstärkers 14 zu erzeugen.

Eine Übertragungsfunktion von der Sendespule 3 zur Empfangsspule 4 ist durch die folgende Formel gegeben:

$$ \frac{I_4}{U_1} = \frac{N_4}{N_1} \frac{1}{Z_A + R_W N_4^2 \left(1 + \dfrac{Z_A}{j\omega L_{44}}\right)} $$

Der Kehrwert der Eingangsimpedanz $Z_E$ also die Eingangsadmittanz $Y_E$ ist durch die folgende Formel gegeben:

$$ Y_E = \frac{1}{N_1^2 R_W + \left(\dfrac{N_1}{N_4}\right)^2 \left(Z_A \| j\omega L_{44}\right)} + \frac{1}{j\omega L_{11}} $$

In den obigen Formeln ist j die imaginäre Einheit, $\omega$ die Kreisfrequenz, $L_{11}$ eine Induktivität zwischen Sendespule 3 und dem Medium 8, $L_{44}$ eine Induktivität zwischen der Empfangsspule 4 und dem Medium 8 und symbolisiert $(Z_A \| j\omega L_{44})$ eine Rechenanweisung zur Berechnung der Impedanz einer Parallelschaltung von $Z_A$ und $j\omega L_{44}$.

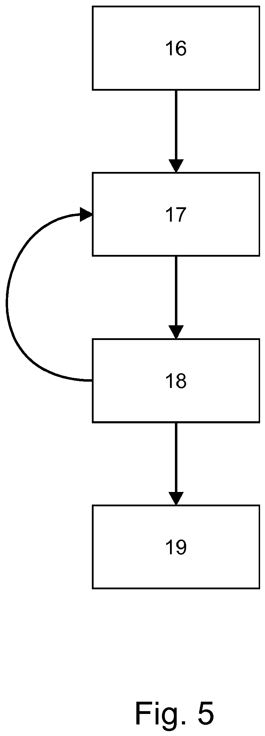

Aus den vorstehenden Formeln ist ersichtlich, dass die Abschlussimpedanz $Z_A$ ein Parameter sowohl der Übertragungsfunktion als auch der Eingangsimpedanz $Z_E$ ist. Somit ist die Eingangsimpedanz $Z_E$ durch Einstellung der Abschlussimpedanz $Z_A$ an die Soll-Eingangsimpedanz anpassbar. Dazu ist die Steuerungseinrichtung 7 zur Ausführung des im Ablaufplan in Fig. 5 dargestellten Verfahrens mit den folgenden Verfahrensschritten ausgebildet.

Im ersten Verfahrensschritt 16 wird eine Soll-Eingangsimpedanz vorgegeben. Bei dieser handelt es sich um die in der Steuerungseinrichtung 7 gespeicherten Soll-Eingangsimpedanz.

Im zweiten Verfahrensschritt 17 wird die Eingangsimpedanz $Z_E$ am Sendespulenanschluss 5 bestimmt.

Im dritten Verfahrensschritt 18 wird die Abschlussimpedanz $Z_A$ derart eingestellt, dass die Eingangsimpedanz $Z_E$ an die Soll-Eingangsimpedanz angepasst ist.

Im vierten Verfahrensschritt 19 wird dann eine Leitfähigkeit des Mediums 8 unter Verwendung der angepassten Eingangsimpedanz $Z_E$ und der eingestellten Abschlussimpedanz $Z_A$ bestimmt. Die Leitfähigkeit wird bestimmt, indem zunächst der Mediumwiderstandswert $R_W$ und dann die Leitfähigkeit des Mediums 8 unter Verwendung des Mediumwiderstandswert $R_W$ und der Geometrie des Mediums 8 zwischen Sendespule 3 und Empfangsspule 4 bestimmt wird.

Dabei werden die aufgeführten Verfahrensschritte nach Bedarf auch mehrfach von der Steuerungseinrichtung 7 ausgeführt. Das gilt insbesondere für den zweiten Verfahrenschritt 17 und den dritten Verfahrensschritt 18, wenn die Eingangsimpedanz $Z_E$ mittels eines iterativen Verfahrens durch Variation der Abschlussimpedanz $Z_A$ an die Soll-Eingangsimpedanz angepasst wird.

**Bezugszeichen**

## EP 3 372 997 B1

| 1 | Induktives Leitfähigkeitsmessgerät |
|---|---|
| 2 | Hohlzylinderförmiger Träger |
| 3 | Sendespule |
| 4 | Empfangsspule |
| 5 | Sendespulenanschluss |
| 6 | Empfangsspulenanschluss |
| 7 | Steuerungseinrichtung |
| 8 | Medium |
| 9 | Sendewechselspannungsquelle |
| 10 | Abschlussimpedanzeinrichtung |
| 11 | Mediumwiderstand mit Mediumwiderstandswert RW |
| 12 | Stromsensor |
| 13 | Gesteuerte Spannungsquelle |
| 14 | Operationsverstärker |
| 15 | Signalgenerator |

| $N_1$ | Anzahl der Windungen der Sendespule |
|---|---|
| $N_4$ | Anzahl der Windungen der Empfangsspule |
| $I_1$ | Sendespulenstrom |
| $I_4$ | Empfangsspulenstrom |
| $L_{11}$ | Induktivität zwischen Sendespule 3 und Medium 8 |
| $L_{44}$ | Induktivität zwischen Empfangsspule 4 und Medium 8 |
| $R_W$ | Mediumwiderstandswert |
| $U_1$ | Sendespulenspannung |
| $U_4$ | Empfangsspulenspannung |
| $Z_A$ | Ausgangsimpedanz |
| $Z_E$ | Eingangsimpedanz |

## Patentansprüche

1. Verfahren zum Betreiben eines induktiven Leitfähigkeitsmessgeräts (1),
   wobei das Leitfähigkeitsmessgerät (1) eine Sendespule (3), eine Empfangsspule (4) und eine Abschlussimpedanzeinrichtung (10) aufweist,
   wobei die Sendespule (3) einen Sendespulenanschluss (5), die Empfangsspule (4) einen Empfangsspulenanschluss (6) und die Abschlussimpedanzeinrichtung (10) eine Abschlussimpedanz ($Z_A$) aufweist,
   wobei die Empfangsspule (4) mit der Abschlussimpedanzeinrichtung (10) abgeschlossen ist und
   wobei die Sendespule (3) und die Empfangsspule (4) durch ein elektrisch leitfähiges Medium (8) induktiv miteinander gekoppelt sind,
   **dadurch gekennzeichnet,**
   **dass** eine Soll-Eingangsimpedanz vorgegeben wird,
   **dass** eine Eingangsimpedanz ($Z_E$) am Sendespulenanschluss (5) bestimmt wird,
   **dass** die Abschlussimpedanz ($Z_A$) derart eingestellt wird, dass die Eingangsimpedanz ($Z_E$) an die Soll-Eingangsimpedanz angepasst ist und
   **dass** eine Leitfähigkeit des Mediums (8) unter Verwendung der angepassten Eingangsimpedanz ($Z_E$)

und der eingestellten Abschlussimpedanz ($Z_A$) bestimmt wird.

2. Verfahren nach Anspruch 1, **dadurch gekennzeichnet, dass** die Eingangsimpedanz ($Z_E$) derart an die Soll-Eingangsimpedanz angepasst wird, dass ein Realteil der Eingangsimpedanz ($Z_E$) einem Realteil der Soll-Eingangsimpedanz entspricht.

3. Verfahren nach Anspruch 1 oder 2, **dadurch gekennzeichnet, dass** die Eingangsimpedanz ($Z_E$) derart an die Soll-Eingangsimpedanz angepasst wird, dass die Eingangsimpedanz ($Z_E$) der Soll-Eingangsimpedanz entspricht.

4. Induktives Leitfähigkeitsmessgerät 1 mit einer Sendespule (3), einer Empfangsspule (4), einer Abschlussimpedanzeinrichtung (10) und einer Steuerungseinrichtung (7),
   wobei die Sendespule (3) einen Sendespulenanschluss (5), die Empfangsspule (4) einen Empfangsspulenanschluss (6) und die Abschlussimpedanzeinrichtung (10) eine Abschlussimpedanz ($Z_A$) aufweist,
   wobei die Empfangsspule (4) mit der Abschlussimpedanzeinrichtung (10) abgeschlossen ist und
   wobei im Betrieb die Sendespule (3) und die Empfangsspule (4) durch ein elektrisch leitfähiges Medium (8) induktiv miteinander gekoppelt sind,
   **dadurch gekennzeichnet,**
   **dass** eine Soll-Eingangsimpedanz in der Steuerungseinrichtung (7) gespeichert ist,
   **dass** die Abschlussimpedanz ($Z_A$) der Abschlussimpedanzeinrichtung (10) einstellbar ist und
   **dass** die Steuerungseinrichtung (7) ausgebildet ist, eine Eingangsimpedanz ($Z_E$) am Sendespulenanschluss (5) zu bestimmen, die Abschlussimpedanz ($Z_A$) derart einzustellen, dass die Eingangsimpedanz ($Z_E$) an die Soll-Eingangsimpedanz angepasst ist und
   eine Leitfähigkeit des Mediums (8) unter Verwendung der angepassten Eingangsimpedanz ($Z_E$) und der eingestellten Abschlussimpedanz zu bestimmen.

5. Induktives Leitfähigkeitsmessgerät (1) nach Anspruch 4, **dadurch gekennzeichnet, dass** die Steuerungseinrichtung (7) ausgebildet ist, die Verfahrensschritte nach Anspruch 2 oder 3 auszuführen.

6. Induktives Leitfähigkeitsmessgerät (1) nach Anspruch 4 oder 5, **dadurch gekennzeichnet,**
   **dass** die Abschlussimpedanzeinrichtung (10) einen Stromsensor (12) zur Messung eines Empfangsspulenstroms ($I_4$) durch den Empfangsspulenanschluss (6) und eine gesteuerte Spannungsquelle (13) zur Einstellung einer Empfangsspulenspannung ($U_4$) über den Empfangsspulenanschluss (6) aufweist

7

und
**dass** die Steuerungseinrichtung (7) ausgebildet ist, den Empfangsspulenstrom ($I_4$) mit dem Stromsensor (12) zu bestimmen und die Empfangsspulenspannung ($U_4$) derart einzustellen, dass die Abschlussimpedanz ($Z_A$) eingestellt ist.

7. Induktives Leitfähigkeitsmessgerät (1) nach Anspruch 6, **dadurch gekennzeichnet, dass** der Stromsensor (12) ein Shunt-Widerstand ist und die gesteuerte Spannungsquelle (13) einen Operationsverstärker (14) zur Bereitstellung der Empfangsspulenspannung ($U_A$) aufweist.

8. Induktives Leitfähigkeitsmessgerät (1) nach Anspruch 7, **dadurch gekennzeichnet, dass** die gesteuerte Spannungsquelle (13) einen Signalgenerator (15) zur Steuerung des Operationsverstärkers (14) aufweist.

9. Induktives Leitfähigkeitsmessgerät (1) nach Anspruch 8, **dadurch gekennzeichnet, dass** der Signalgenerator (15) ausgebildet ist, ein wertkontinuierliches Signal oder ein pulsweitenmoduliertes Signal zur Ansteuerung des Operationsverstärkers (14) zu erzeugen.

**Claims**

1. Method for operating an inductive conductivity meter (1),
   wherein the conductivity meter (1) comprises a transmitting coil (3), a receiving coil (4) and a terminating impedance device (10),
   wherein the transmitting coil (3) has a transmitting coil terminal (5), the receiving coil (4) has a receiving coil terminal (6) and the terminating impedance device (10) has a terminating impedance ($Z_A$),
   wherein the receiving coil (4) is terminated with the terminating impedance device (10) and
   wherein the transmitting coil (3) and the receiving coil (4) are inductively coupled with one another by an electrically conductive medium (8),
   **characterized in**
   **that** a setpoint input impedance is specified,
   an input impedance ($Z_E$) is determined at the transmitting coil terminal (5),
   **that** the terminating impedance ($Z_A$) is set such that the input impedance ($Z_E$) is matched to the setpoint input impedance, and
   **that** a conductivity of the medium (8) is determined using the adjusted input impedance ($Z_E$) and the set termination impedance ($Z_A$).

2. Method according to claim 1, **characterized in that** the input impedance ($Z_E$) is matched to the setpoint input impedance such that a real part of the input impedance ($Z_E$) corresponds to a real part of the setpoint input impedance.

3. Method according to claim 1 or 2, **characterized in that** the input impedance ($Z_E$) is matched to the setpoint input impedance such that the input impedance ($Z_E$) corresponds to the setpoint input impedance.

4. Inductive conductivity meter (1) with a transmitting coil (3), a receiving coil (4), a terminating impedance device (10) and a control device (7),
   wherein the transmitting coil (3) has a transmitting coil terminal (5), the receiving coil (4) has a receiving coil terminal (6) and the terminating impedance device (10) has a terminating impedance ($Z_A$),
   wherein the receiving coil (4) is terminated with the terminating impedance device (10) and
   wherein, during operation, the transmitting coil (3) and the receiving coil (4) are inductively coupled with one another by an electrically conductive medium (8),
   **characterized in**
   **that** a setpoint input impedance is stored in the control device (7),
   **that** the terminating impedance ($Z_A$) of the terminating impedance device (10) is adjustable, and
   **that** the control device (7) is designed
   to determine an input impedance ($Z_E$) at the transmitting coil terminal (5),
   to set the terminating impedance ($Z_A$) such that the input impedance ($Z_E$) is matched to the setpoint input impedance, and
   to determine a conductivity of the medium (8) using the adjusted input impedance ($Z_E$) and the set terminating impedance.

5. Inductive conductivity meter (1) according to claim 4, **characterized in that** the control device (7) is designed for implementing the method steps according to claim 2 or 3.

6. Inductive conductivity meter (1) according to claim 4 or 5, **characterized in**
   **that** the terminating impedance device (10) has a current sensor (12) for measuring a receiving coil current ($I_4$) through the receiving coil terminal (6) and a controlled voltage source (13) for setting a receiving coil voltage ($U_4$) across the receiving coil terminal (6), and
   **that** the control device (7) is designed to determine the receiving coil current ($I_4$) with the current sensor (12) and to set the receiving coil voltage ($U_4$) such that the terminating impedance ($Z_A$) is set.

7. Inductive conductivity meter (1) according to claim 6, **characterized in that** the current sensor (12) is a shunt resistor and the controlled voltage source (13) has an operational amplifier (14) for providing the receiving coil voltage ($U_A$).

8. Inductive conductivity meter (1) according to claim 7, **characterized in that** the controlled voltage source (13) has a signal generator (15) for controlling the operational amplifier (14).

9. Inductive conductivity meter (1) according to claim 8, **characterized in that** the signal generator (15) is designed to generate a continuous-value signal or a pulse width modulated signal for controlling the operational amplifier (14).

**Revendications**

1. Procédé de fonctionnement d'un conductimètre inductif (1),
le conductimètre (1) comprenant une bobine émettrice (3), une bobine réceptrice (4) et un dispositif à impédance de terminaison (10),
la bobine émettrice (3) comportant une borne de bobine émettrice (5), la bobine réceptrice (4) comportant une borne de bobine réceptrice (6) et le dispositif à impédance de terminaison (10) comportant une impédance de terminaison ($Z_A$),
la bobine réceptrice (4) étant terminée par le dispositif à impédance de terminaison (10) et
la bobine émettrice (3) et la bobine réceptrice (4) étant couplées l'une à l'autre de manière inductive par un milieu électriquement conducteur (8),
**caractérisé en ce que**
une impédance d'entrée de consigne est spécifiée,
une impédance d'entrée ($Z_E$) est déterminée au niveau de la borne de bobine émettrice (5),
l'impédance de terminaison ($Z_A$) est réglée de telle sorte que l'impédance d'entrée ($Z_E$) soit adaptée à l'impédance d'entrée de consigne et
une conductivité du milieu (8) est déterminée à l'aide de l'impédance d'entrée ($Z_E$) adaptée et l'impédance de terminaison ($Z_A$) définie.

2. Procédé selon la revendication 1, **caractérisé en ce que** l'impédance d'entrée ($Z_E$) est adaptée à l'impédance d'entrée de consigne de sorte qu'une partie réelle de l'impédance d'entrée ($Z_E$) correspond à une partie réelle de l'impédance d'entrée de consigne.

3. Procédé selon la revendication 1 ou 2, **caractérisé en ce que** l'impédance d'entrée ($Z_E$) est adaptée à l'impédance d'entrée de consigne de sorte que l'impédance d'entrée ($Z_E$) corresponde à l'impédance d'entrée de consigne.

4. Conductimètre inductif 1 comprenant une bobine émettrice (3), une bobine réceptrice (4), un dispositif à impédance de terminaison (10) et un dispositif de commande (7),
la bobine émettrice (3) comportant une borne de bobine émettrice (5), la bobine réceptrice (4) comportant une borne de bobine réceptrice (6) et le dispositif à impédance de terminaison (10) comportant une impédance de terminaison ($Z_A$),
la bobine réceptrice (4) étant terminée par le dispositif à impédance de terminaison (10) et
la bobine émettrice (3) et la bobine réceptrice (4) étant couplées, en fonctionnement, l'une à l'autre de manière inductive par un milieu électriquement conducteur (8),
**caractérisé en ce que**
une impédance d'entrée de consigne est mémorisée dans le dispositif de commande (7),
l'impédance de terminaison ($Z_A$) du dispositif à impédance de terminaison (10) est réglable et
le dispositif de commande (7) est conçu pour déterminer une impédance d'entrée ($Z_E$) au niveau de la borne de bobine émettrice (5),
régler l'impédance de terminaison ($Z_A$) de sorte que l'impédance d'entrée ($Z_E$) soit adaptée à l'impédance d'entrée de consigne, et
déterminer une conductivité du milieu (8) à l'aide de l'impédance d'entrée ($Z_E$) adaptée et l'impédance de terminaison réglée.

5. Conductimètre inductif (1) selon la revendication 4, **caractérisé en ce que** le dispositif de commande (7) est conçu pour effectuer les étapes de procédé selon la revendication 2 ou 3.

6. Conductimètre inductif (1) selon la revendication 4 ou 5, **caractérisé en ce que**
le dispositif à impédance de terminaison (10) comporte un capteur de courant (12) destiné à mesurer un courant de bobine réceptrice ($I_4$) par le biais de la borne de bobine réceptrice (6) et une source de tension commandée (13) destinée à régler une tension de bobine réceptrice ($U_4$) par le biais de la borne de bobine réceptrice (6), et
le dispositif de commande (7) est conçu pour déterminer le courant de bobine réceptrice ($I_4$) avec le capteur de courant (12) et pour régler la tension de bobine réceptrice ($U_4$) de manière à régler l'impédance de terminaison ($Z_A$).

7. Conductimètre inductif (1) selon la revendication 6, **caractérisé en ce que** le capteur de courant (12) est une résistance parallèle et la source de tension commandée (13) comporte un amplificateur opérationnel (14) destiné à produire la tension de bobine réceptrice ($U_A$).

8. Conductimètre inductif (1) selon la revendication 7, **caractérisé en ce que** la source de tension commandée (13) comprend un générateur de signal (15) destiné à commander l'amplificateur opérationnel (14).

9. Conductimètre inductif (1) selon la revendication 8, **caractérisé en ce que** le générateur de signal (15) est conçu pour générer un signal à valeur continue ou un signal modulé en largeur d'impulsion pour commander l'amplificateur opérationnel (14).

1

7

5   6

2   8

3

4

Fig. 1

Fig. 2

Fig. 3

Fig. 4

```
┌─────────────┐
│     16      │
└─────────────┘
       │
       ▼
┌─────────────┐
│     17      │◄──┐
└─────────────┘   │
       │          │
       ▼          │
┌─────────────┐   │
│     18      │───┘
└─────────────┘
       │
       ▼
┌─────────────┐
│     19      │
└─────────────┘
```

Fig. 5

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 3491287 A
- DE 102009026403 A1
- GB 1085557 A